# EUROPEAN PATENT APPLICATION

(11) **EP 3 081 212 A1**
(43) Date of publication of application: **19.10.2016**
(21) Application number: 15163871.5
(22) Date of filing: 16.04.2015
(51) Int. Cl.: A61K 31/198, A61Q 19/00, A61P 17/02

(54) **CARBOXYMETHYLCYSTEINE FOR TOPICAL TREATMENT OF STRETCH MARKS**

(71) Applicant: Polichem SA, 1526 Luxembourg (LU)
(72) Inventor: Mailland, Federico, CH-6900 Lugano (CH); Scarci, Francesco, I-22100 Como (IT); Iob, Giuliana, CH-6953 Lugaggia (CH)
(74) Representative: Pistolesi, Roberto

(57) **Abstract**

Carboxymethylcysteine and pharmaceutically acceptable salts thereof improve the appearance and the structure of skin affected by stretch marks due to either pregnancy, weight training, weight gain or adolescent growth. Carboxymethylcysteine is preferably formulated in an appropriate topical composition and may be applied directly on the stretch marks; the composition may have a content in carboxymethylcysteine or a pharmaceutically acceptable salt from 0.1 to 25 % by weight.

## Description

The present invention relates to the use of carboxymethylcysteine and/or pharmaceutically acceptable salts thereof to improve the appearance and the structure of skin affected by stretch marks due to either pregnancy, weight training, weight gain or adolescent growth. Carboxymethylcysteine is preferably formulated in an appropriate topical composition and may be applied directly on the stretch marks.

### BACKGROUND

Stretch marks are a disfiguring condition associated with rapid stretching of the skin, as occurs for example with the abdominal enlargement of pregnancy. They can be found in children who have become rapidly obese and during the rapid growth of puberty in males and females. They are common in adult obese women and are mainly located on the breasts, hips, thighs, buttocks, abdomen, and flank. Stretch marks appear as parallel streaks of red, thinned, glossy skin that over time become whitish and scar-like in appearance. The stretch marks may be slightly depressed and have a different texture than normal skin. They are virtually impossible to completely and permanently remove. This is because they are the marks of damaged fibers under the skin. The pathogenesis is unknown but probably relates to changes in those structures that provide skin with its tensile strength. The whole process is triggered by ongoing inflammation processes in the dermis. The actual stretch mark is the consequence of the dermis stretching to the point of breaking cell-to-cell contact and the fibers of the basal membrane. The dermal collagen and elastin fiber-matrix are damaged and dehydrated leaving visible marks or scars (Mitts TF, Jimenez F, Hinek A.: Skin biopsy analysis reveals predisposition to stretch mark formation. Aesthet Surg J. 2005 Nov-Dec;25(6):593-600). Though stretch marks can rarely be removed completely, there are many treatments available for the purpose of improving the appearance of existing stretch marks including cosmetic surgery (Lemperle G, Tenenhaus M, Knapp D, Lemperle SM. The direction of optimal skin incisions derived from striae distensae. Plast Reconstr Surg. 2014 Dec;134(6):1424-34), laser treatments (de Angelis F, Kolesnikova L, Renato F, Liguori G. Fractional nonablative 1540-nm laser treatment of striae distensae in Fitzpatrick skin types II to IV: clinical and histological results. Aesthet Surg J. 2011 May;31(4):411-9; Güngör S, Sayilgan T, Gökdemir G, Ozcan D. Evaluation of an ablative and non-ablative laser procedure in the treatment of striae distensae. Indian J Dermatol Venereol Leprol. 2014 Sep-Oct;80(5):409-12) and dermabrasion (Hexsel D, Soirefmann M, Porto MD, Schilling-Souza J, Siega C, Dal Forno T. Superficial dermabrasion versus topical tretinoin on early striae distensae: a randomized, pilot study. Dermatol Surg. 2014 May;40(5):537-44). However, these treatments are rather costly and downright painful for some, with no assurance that it will indeed work.

Topical preparations of creams or gels have also been used with controversial results. Some efficacy has been reported by use of tretinoin or other retinoids, but their use should be avoided in women of childbearing potential due to possible absorption and teratogenic effects (Sarnoff DS. Therapeutic Update on the Treatment of Striae Distensae. Drugs Dermatol. 2015 Jan 1;14(1):11-12). Keratolytic and exfoliants have also been reported as useful due their peeling effect. Those products included salicylic and glycolic acid (Ash K, Lord J, Zukowski M, McDaniel DH. Comparison of topical therapy for striae alba - 20% glycolic acid/0.05% tretinoin versus 20% glycolic acid/10% L-ascorbic acid -. Dermatol Surg. 1998 Aug; 24(8):849-56). The massage with a silicone gel over a period of 6 weeks increased significantly the content of collagen in stretch marks over placebo, and decreased pliability (Ud-Din S, McAnelly SL, Bowring A. et al, A double-blind controlled clinical trial assessing the effect of topical gels on striae distensae (stretch marks): a non-invasive imaging, morphological and immunohistochemical study. Arch Dermatol Res (2013) 305:603-617). Most of the above products may cause irritation and efficacy remains controversial (Brennan M, Young G, Devane D. Topical preparations for preventing stretch marks in pregnancy. Cochrane Database Syst Rev. 2012 Nov 14;11:CD000066).

Thus, there is still an important medical need for topical preparations which can be effectively and safely used for minimizing stretch marks.

Carboxymethylcysteine (carbocisteine) is a well-known mucolytic agent that reduces the viscosity of sputum and can be used to help relieve the symptoms of chronic obstructive pulmonary disorder (COPD) and bronchiectasis by allowing patients to bring up sputum more easily. This compound and its salts are known in the art (CN1266681) to promote healing of wound and bums when combined with sodium citrate, sodium laurylsulfate, magnesium laurylsulfate and sodium deoxycholate. WO2008017521A2 discloses using at least a thiolated compound, such as carboxymethylcysteine, and at least an ester of an organic acid for improving the elasticity of the vagina and of the perineum in pregnant women.

### DEFINITIONS

Unless otherwise defined, all terms of art, notations and other scientific terminology used herein are intended to have the meanings commonly understood by those persons skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "physiologically acceptable excipient" herein refers to a substance devoid of any pharmacological effect of its own and which does not produce adverse reactions when administered to a mammal, preferably a human. Physiologically acceptable excipients are well known in the art and are disclosed, for instance in the Handbook of Pharmaceutical Excipients, sixth edition 2009, herein incorporated by reference.

The term "pharmaceutically acceptable salts" herein refers to those salts or derivatives which possess the biological effectiveness and properties of the salified or derivatized compound and which do not produce adverse reactions when administered to a mammal, preferably a human. The pharmaceutically acceptable salts may be inorganic or organic salts; examples of pharmaceutically acceptable salts include but are not limited to: sodium, potassium, calcium, ammonium, lysine, arginine.

The terms "approximately" and "about" herein refer to the range of the experimental error, which may occur in a measurement.

The terms "comprising", "having", "including" and "containing" are to be construed as openended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as a semi-closed terms, meaning that no other ingredients which materially affects the basic and novel characteristics of the invention are included (optional excipients may thus be included).

The terms "consists of", "consisting of" are to be construed as a closed term.

The term "oil-gel" herein refers to an anhydrous oil jellified with a polymer.

The term "oil-bath solution" herein refers to an oil and one or more emulsifiers concentrate for extemporaneous emulsion when put in the bath.

### DISCLOSURE OF THE INVENTION

It has now surprisingly been found that carboxymethylcysteine and/or pharmaceutically acceptable salts thereof, besides being able to soften protein substrates in the mucus and in the skin, are active in scar repair and may be useful to improve the appearance and the structure of the skin affected by stretch marks. The regular use of carboxymethylcysteine formulated in an appropriate composition suitable for topical application directly on the stretch marks is able to decrease the thickness, the relief and the pliability of the skin lesions, thus improving both the structure and the appearance of the concerned skin areas. Moreover, the beneficial effect of carboxymethylcysteine is further increased by the presence of petrolatum (vaseline) in the composition. The two components act synergistically in repairing the collagen portion of the skin matrix from fibroblasts, thus increasing the benefit for the patient by application of compositions containing simultaneously carboxymethylcysteine and petrolatum.

An embodiment of the present invention is therefore the use of carboxymethylcysteine and/or a pharmaceutically acceptable salts thereof in the treatment of stretch marks. Preferably, said pharmaceutically acceptable salt is the lysine salt.

In an embodiment said stretch marks are located in any part of body or legs. In a further preferred embodiment the stretch marks are due to post-pregnancy, weight training, weight gain or adolescent growth.

In a further preferred embodiment, carboxymethylcysteine or a pharmaceutically acceptable salt thereof is used in combination with petrolatum as emollient.

In a further embodiment, carboxymethylcysteine or a pharmaceutically acceptable salt thereof, either alone or in combination with petrolatum, is administered in the form of a pharmaceutical formulation. In a further embodiment, the pharmaceutical formulation is administered topically. Preferably, said pharmaceutical formulation is in form of ointment, emulsion, cream, lotion, stick, massage oil-gel, bath-oil solution, and is preferably topically applied by gently massage over the stretch marks for a few minutes, to improve penetration of the ingredients deeply into the scar tissues.

In a further embodiment said pharmaceutical formulation has a content in carboxymethylcysteine and/or a pharmaceutically acceptable salt thereof from 0.1 to 25 % by weight, preferably from 0.5 to 15 % by weight, more preferably from 1.0 to 10 % by weight, with respect to the total weight of the formulation.

In a preferred embodiment, the pharmaceutical formulation contains carboxymethylcysteine in form of lysine salt.

In a further embodiment, said pharmaceutical formulation has a content in petrolatum from 1 to 35 % by weight, preferably from 2.5 to 25 % by weight, more preferably from 5 to 20% by weight, with respect to the total weight of the formulation

The pharmaceutical formulation of the present invention may include physiologically acceptable excipients selected from emollients, rheological additives, thickening agents, emulsifiers, humectants, buffering agents, antioxidant agents, chelating agents, moisturizing agents, exfoliating agents, decongestant agents, disinfectant and/or antimicrobial agents, flavoring and colorants.

The preferred suitable emollients include oils or other fatty materials, such as prunus amygdalus dulcis oil, petrolatum, paraffinum liquidum, hydrogenated castor oil, hydrogenated polydecene, octyldodecanol, cera alba, cetostearyl alcohol, sesamum indicum seed oil, dicaprylyl carbonate, isodecylneopentanoate, C10-18 triglycerides, silicones, such as dimethicone or cyclopenthasiloxane.

The preferred suitable rheological additives include xanthan gum, carbomer or ammonium acryloyldimethyltaurate/VP copolymer (copolymer of ammonium acryloyldimethyltaurate and vinylpyrrolidone monomers).

The preferred suitable thickening agents include silica or polyethylene.

The preferred suitable emulsifiers include PEG-100 stearate, glyceryl stearate, polyglyceryl-3 methylglucose distearate, olivoyl hydrolyzed wheat protein, glyceryl oleate, polysorbate 65 or sucrose palmitate.

The preferred suitable humectants include propylene glycol, glycerin or propanediol.

In a further embodiment of the present invention, carboxymethylcysteine and/or a pharmaceutically acceptable salt thereof may be used as the only active principle, or it may be used in combination with one or more active principles such as allantoin, panthenol, betaine, tocopheryl acetate, tocopherol or lactic acid. According to an embodiment, the formulation consists of or essentially consists of carboxymethylcysteine and/or a pharmaceutically acceptable salt thereof, one or more physiologically acceptable excipients and optionally one or more active principles selected from allantoin, panthenol, betaine, tocopheryl acetate, tocopherol or lactic acid.

The pharmaceutical formulation will be prepared according to conventional techniques. Examples of pharmaceutical formulations prepared according to the present invention include: ointment, emulsion, cream, lotion, stick, massage oil-gel, bath-oil solution.

The pharmaceutical formulations and the uses of the present invention will now be more fully described by the following examples. It should, however, be noted that such examples are given by way of illustration and not of limitation.

### EXAMPLES

### Example 1

An ointment, having the following w/w % composition, is prepared:

| | | |
|---|---|---|
| 1) | Lysine Carboxymethyl Cysteinate¹ | 5.00% |
| 2) | Polyglyceryl-3 Beeswax | 7.50% |
| 3) | Prunus Amygdalus Dulcis | 12.00% |
| 4) | Tocopherol, Lecithin, Ascorbyl palmitate, Citric acid² | 0.05% |
| 5) | Petrolatum | 15.00% |
| 6) | Hydrogenated Castor Oil | 2.00% |
| 7) | Silica | 1.50% |
| 8) | Paraffinum Liquidum | q.s. to 100.00% |

| | | |
|---|---|---|
| ¹Elastocell^{®} (Lysine Carboxymethyl Cysteinate 30% water solution). ²Aperoxid TLA. | | |

### Preparation:

Paraffinum Liquidum, Polyglyceryl-3 Beeswax, Prunus Amygdalus Dulcis, Aperoxid TLA Petrolatum and Hydrogenated Castor Oil are mixed and heated at 75 °C - 80°C. When the mass is melted, silica is added under stirring. The mass is mixed to obtain an homogeneous dispersion.

The melted mass is then cooled at 45°C and Lysine Carboxymethyl Cysteinate is added under vigorous stirring. The resulting mass is cooled to room temperature under continuous mixing and an homogeneous ointment is obtained.

### Example 2

A clinical study was performed to assess the aesthetic efficacy of the composition according to the Example 1 on reducing stretch marks on the body and legs of 33 women. The study followed a randomized, open-label, blind-evaluator, intra-individual comparison split-body design. Female subjects aged from 18 to 65 years with stable weight for at least 2 months signing the informed consent form were included. They were characterized by stretch marks on the body/legs due to post pregnancy, weight training, weight gain or adolescent growth. All included women presented two comparable areas with stretch marks on the body/legs or one large area where to allocate the test and the control area. The composition according to the Example 1 (Prd B) was applied twice a day by a gently massage of 5 minutes on the test area and compared to the control (untreated) area (Prd A) over a period of 8 weeks. Test and control area (5x5 cm size) were randomized.

The results were evaluated by means of the OSAS (= Observer Scar Assessment Scale). This scale is considering 6 items, namely vascularity, pigmentation, thickness, relief, pliability and surface area of the skin lesions, in a 10-point score, where 0= no lesion and 10= maximum imaginable lesion. For Prd B there was a significant decrease of mean thickness from 4.1 at day 1 to 3.6 at day 57, no change for Prd A (p=0.003 between Prd B and A); mean relief decreased from 4.2 at day 1 to 3.8 at day 57 for Prd B, no change for A (p=0.038); average pliability decreased from 5.2 to 4.4 for Prd B, no change for A (p<0.001). No significant differences were found in vascularity, pigmentation or surface area. The overall OSAS score decreased from 23.8 at day 1 to 22.0 at day 57 for Prd B, while for Prd A it passed from 23.3 at day 1 to 23.6 at day 57 (p<0.001 between Prd B and A).

In conclusion, the data of this study put in evidence an effect of the composition according to the Example 1 to decrease severity of stretch marks when regularly applied for over 8 weeks on the affected skin.

### Example 3

A soft emulsion (cream), having the following w/w % composition, is prepared:

| | | |
|---|---|---|
| 1) | Purified water | q.s. to 100% |
| 2) | EDTA | 0.10% |
| 3) | Betaine | 2.00% |
| 4) | Allantoin | 0.15% |
| 5) | Propylene Glycol | 5.00% |
| 6) | Ammonium Acryloyldimethyltaurate/VP Copolymer | 0.90% |
| 7) | Peg-100 Stearate, Glyceryl Stearate | 3.00% |
| 8) | Cetostearyl Alcohol | 1.00% |
| 9) | Hydrogenated Polydecene | 6.00% |
| 10) | Octyldodecanol | 2.00% |
| 11) | Petrolatum | 7.00% |
| 12) | Cera Alba | 1.00% |
| 13) | Dimethicone | 1.00% |
| 14) | Tocopherol | 0.50% |
| 15) | Cyclopentasiloxane | 3.00% |
| 16) | Lysine Carboxymethyl Cysteinate² | 17.00% |
| 17) | Preservative | q.s. |
| 18) | Buffer (Lactic Acid, Sodium Hydroxide) | q.s. |
| 19) | Parfum | q.s. |

| | | |
|---|---|---|
| ² Elastocell® (Lysine Carboxymethyl Cysteinate 30% water solution). | | |

### Preparation:

In the main vessel, solubilize components 2, 3, 4, 5 in part of water, stirring after each addition. Add component 6 and disperse it thoroughly until homogeneity. Heat at 70 - 75°C.

In another vessel combine components 7, 8, 9, 10, 11, 12, 13, 14 and heat at 70°C -75°C under moderate stir. Add the lipophilic phase to the main vessel and homogenize for about 10 minutes.

Cool down to 40°C and add on sequence components 15, 16, 17, 18 and 19, homogenizing after each addition for about 3 - 5 minutes.

Cool down to room temperature under moderate stirring.

### Example 4

A liquid emulsion (lotion), having the following w/w % composition, is prepared:

| | | |
|---|---|---|
| 1) | Purified water | q.s. to 100% |
| 2) | EDTA | 0.10% |
| 3) | Panthenol | 0.50% |
| 4) | Allantoin | 0.15% |
| 5) | Propanediol | 3.00% |
| 6) | Xanthan Gum | 0.20% |
| 7) | Carbomer | 0.15% |
| 8) | Polyglyceryl-3 Methylglucose Distearate | 1.50% |
| 9) | Olivoyl Hydrolyzed Wheat Protein, Cetearyl Alcohol, Glyceryl Oleate, Glyceryl Stearate, Potassium Hydroxide¹ | 4.50% |
| 10) | Sesamum Indicum Seed Oil | 4.00% |
| 11) | Dicaprylyl Carbonate | 3.00% |
| 12) | Isodecyl Neopentanoate | 2.00% |
| 13) | Petrolatum | 5.00% |
| 14) | Tocopheryl Acetate 0.50% | |
| 15) | Lysine Carboxymethyl Cysteinate² | 33.50% |
| 16) | Buffer (Lactic Acid, Sodium Hydroxide) | q.s. |
| 17) | Preservative | q.s. |
| 18) | Parfum | q.s. |

| | | |
|---|---|---|
| ¹ Olivoil Emulsifier (Kalichem). ²Elastocell® (Lysine Carboxymethyl Cysteinate 30% water solution). | | |

### Preparation:

In the main vessel, solubilize components 2, 3, 4, 5 in part of water, stirring after each addition. Add component 6 and disperse it thoroughly until homogeneity. Add component 7 and disperse it thoroughly using an homogenizer. Heat at 70 - 75°C. In another vessel combine components 8, 9, 10, 11, 12, 13, 14 and heat at 70°C -75°C under moderate stir. Add the lipophilic phase to the main vessel and homogenize for about 10 minutes.

Cool down to 40°C and add on sequence components 15, 16, 17 and 18, homogenizing after each addition for about 3 - 5 minutes.

Cool down to room temperature under moderate stirring.

### Example 5

A stick (solid vehicle), having the following w/w % composition, is prepared:

| | | |
|---|---|---|
| 1) | C10-18 Triglycerides | 85.00% |
| 2) | Polysorbate 65 | 6.00% |
| 3) | Tocopheryl Acetate | 0.50% |
| 4) | Lysine Carboxymethyl Cysteinate¹ | 3.50% |
| 5) | Petrolatum | 5.00% |

| | | |
|---|---|---|
| ¹Elastocell® (Lysine Carboxymethyl Cysteinate 30% water solution). | | |

### Preparation:

Combine components 1, 2, 3, 5 in a melting pot vessel and melt at 65°C - 70°C while stirring; mix vigorously to homogeneity; cool down slowly while mixing at 45°C and add component 4, mixing at this temperature for about 5 - 10 minutes; stop the heating and continue mixing until to reach 35°C - 38C; pour the blend into the mould; start cooling at 5°C for about 10 minutes.

### Example 6

A massage oil-gel, having the following w/w % composition, is prepared:

| | | |
|---|---|---|
| 1) | Paraffinum Liquidum, Polyethylene¹ | 76.00% |
| 2) | Petrolatum | 20.00% |
| 3) | Lysine Carboxymethyl Cysteinate² | 4.00% |

| | | |
|---|---|---|
| ¹Pionier PLW (Hansen & Rosenthal) ²Elastocell® (Lysine Carboxymethyl Cysteinate 30% water solution). | | |

### Preparation:

Combine components 2 and 3. Mix until an homogeneous mass is obtained. Add to component 1 and mix until an homogeneous gel is obtained.

### Example 7

A bath-oil solution, having the following w/w % composition, is prepared:

| | | |
|---|---|---|
| 1) | Sucrose Palmitate | 4.00% |
| 2) | Glycerin | 45.00% |
| 3) | Purified Water | 7.00% |
| 4) | Petrolatum | 5.00% |
| 5) | Hydrogenated Polydecene | 20.00% |
| 6) | Prunus Amygdalus Dulcis Oil | 15.00% |
| 7) | Lysine Carboxymethyl Cysteinate¹ | 3.50% |
| 8) | Parfum | 0.50% |

| | | |
|---|---|---|
| ¹Elastocell® (Lysine Carboxymethyl Cysteinate 30% water solution). | | |

### Preparation:

In the main vessel combine components 1, 2, 3 and heat at 80°C while mixing; in a separate vessel combine components 4, 5, 6 and heat at 80°C. Add to the main vessel very slowly under stirring. Continue mixing until to reach 45°C and add components 7 and 8; mix until room temperature.

## Claims

1. Carboxymethylcysteine and/or a pharmaceutically acceptable salt thereof, for use in the treatment of stretch marks.

2. Carboxymethylcysteine and/or a pharmaceutically acceptable salt thereof for use according to claim 1, **characterized in that** said stretch marks are located in any part of body or legs.

3. Carboxymethylcysteine and/or a pharmaceutically acceptable salt thereof for use according to claim 1 or 2, **characterized in that** said stretch marks are due to post pregnancy, weight training, weight gain or adolescent growth.

4. Carboxymethylcysteine and/or a pharmaceutically acceptable salt thereof for use according to claim 1, **characterized in that** said pharmaceutically acceptable salt is the lysine salt.

5. Carboxymethylcysteine and/or a pharmaceutically acceptable salt thereof for use according to any of the preceding claims, **characterized in that** it is used in combination with petrolatum.

6. Carboxymethylcysteine and/or a pharmaceutically acceptable salt thereof for use according to any of the preceding claims, **characterized in that** it is administered in the form of a pharmaceutical topical formulation, which is preferably an ointment, emulsion, cream, lotion, oil-gel, stick, massage oil-gel or bath-oil solution.

7. Carboxymethylcysteine and/or a pharmaceutically acceptable salt thereof for use according to claim 6, **characterized in that** said pharmaceutical formulation has a content in carboxymethylcysteine and/or a pharmaceutically acceptable salt thereof from 0.1 to 25 % by weight.

8. Carboxymethylcysteine and/or a pharmaceutically acceptable salt thereof for use according to claim 6, **characterized in that** said pharmaceutical formulation has a content in carboxymethylcysteine or a salt thereof from 0.5 to 15 % by weight.

9. Carboxymethylcysteine and/or a pharmaceutically acceptable salt thereof for use according to claim 6, **characterized in that** said pharmaceutical formulation has a content in carboxymethylcysteine or a salt thereof from 1.0 to 10% by weight.

10. Carboxymethylcysteine and/or a pharmaceutically acceptable salt thereof for use according to claim 6, **characterized in that** said pharmaceutical formulation has a content in petrolatum from 1 to 35 % by weight.

11. Carboxymethylcysteine and/or a pharmaceutically acceptable salt thereof for use according to claim 6, **characterized in that** said pharmaceutical formulation has a content in petrolatum from 2.5 to 25 % by weight.

12. Carboxymethylcysteine and/or a pharmaceutically acceptable salt thereof for use according to claim 6, **characterized in that** said pharmaceutical formulation has a content in petrolatum from 5 to 20% by weight.

13. Carboxymethylcysteine and/or a pharmaceutically acceptable salt thereof for use according to claim 6, **characterized in that** said pharmaceutical formulation contains one or more physiologically acceptable excipients.

14. Carboxymethylcysteine and/or a physiologically acceptable salt thereof for use according to claim 13, **characterized in that** said pharmaceutically acceptable excipients are selected from emollients, rheological additives, thickening agents, emulsifiers, humectants, buffering agents, antioxidant agents, chelating agents, moisturizing agents, exfoliating agents, decongestant agents, disinfectant and/or antimicrobial agents, flavoring and colorants.
